# EUROPEAN PATENT APPLICATION

(11) **EP 3 770 181 A1**
(43) Date of publication of application: **27.01.2021**
(21) Application number: 18910697.4
(22) Date of filing: 23.03.2018
(51) Int. Cl.: C08B 37/08, C08J 3/075, C08J 3/24, C08J 3/28, C08F 299/00, C08F 2/48, A61L 27/52

(54) **METHOD FOR PREPARING HYALURONIC ACID HYDROGEL MIROPARTICLES AND USE THEREOF IN REPAIRING ARTICULAR CARTILAGE DEFECTS**

(71) Applicant: Kaohsiung Medical University, Kaohsiung City 807 (TW)
(72) Inventor: HO, Mei-Ling, Taiwan 807 (TW); CHANG, Je-Ken, Taiwan 807 (TW); CHEN, Chung-Hwan, Taiwan 807 (TW); CHEN, Hui-Ting, Taiwan 807 (CN); WU, Shun Cheng, Taiwan 807 (CN); TEONG, Benjamin, Taiwan 807 (CN)
(74) Representative: Lang, Christian
(86) International application number: PCT/CN2018/080135
(87) International publication number: WO 2019/178825

(57) **Abstract**

A method for preparing hyaluronic acid hydrogel microparticles and a use thereof in repairing articular cartilage defects, the method for preparing hyaluronic acid hydrogel microparticles includes: (a) reacting hyaluronic acid with methacrylic anhydride to synthesize a methacrylated hyaluronic acid conjugate; (b) mixing the methacrylated hyaluronic acid conjugate with a photoinitiator, and irradiating ultraviolet light to carry out a photopolymerization reaction so as to obtain a hyaluronic acid hydrogel; and (c) passing the hyaluronic acid hydrogel through a sieve to obtain hyaluronic acid hydrogel microparticles.

## Description

### FIELD OF THE INVENTION

The present invention relates to a method for preparing hyaluronic acid hydrogel microparticles and use thereof in repairing articular cartilage defects.

### BACKGROUND OF THE INVENTION

A cartilage tissue is a connective tissue without blood vessels, lymphatic systems and nerves, and is mainly composed of hyaline cartilage. The hyaline cartilage is mainly composed of chondrocytes, type II collagen and proteoglycan. Once the cartilage tissue is damaged, the number of adjacent chondrocytes is very limited, which is not enough to repair the damage, let alone the problem of being restricted by the coating of the extracellular matrix and difficult to migrate to the injured site. Currently, it is known that the new tissue produced by cartilage self-repair is mostly composed of fibrocartilage tissue, which is mainly type I collagen. Because the fibrocartilage tissue lacks the required biomechanical properties of cartilage and does not have the hyaline cartilage function, it will be subjected to gradual degradation, and it is difficult to restore the joint to the normal activity before injury. Although there are surgical approaches such as microfracture, osteochondral grafting, or autologous chondrocyte implantation in clinics, there are still problems, such as production of fibrocartilage, failure of adherence of newly generated cartilage tissues and chondrocyte degeneration. In recent years, the use of tissue engineering for cartilage tissue repair has developed rapidly. This method is to use an active cellular scaffold with cells such as chondrocytes or mesenchymal stem cells to repair cartilage.

The hyaluronic acid is one of the components of the articular cartilage, and commonly used as a cellular scaffold in articular cartilage repair. When hyaluronic acid is used to repair articular cartilage tissue, it is necessary that chondrocytes or mesenchymal stem cells are used together. Hyaluronic acid together with the cells have the effect of cartilage repair. However, a problem of using the chondrocytes or mesenchymal stem cells is that the cell proliferation is not easy, the cell sources are difficult to control, or allotransplantation may carry pathogens.

How to use hyaluronic acid alone as a cellular scaffold without using the cells is a problem that needs to be solved.

### SUMMARY OF THE INVENTION

The present invention provides a method for preparing hyaluronic acid hydrogel microparticles, comprising: (a) reacting hyaluronic acid with methacrylic anhydride to synthesize a methacrylated hyaluronic acid conjugate; (b) mixing the methacrylated hyaluronic acid conjugate with a photoinitiator, and irradiating ultraviolet light to carry out a photopolymerization reaction to obtain a hyaluronic acid hydrogel; and (c) passing the hyaluronic acid hydrogel through a sieve to obtain the hyaluronic acid hydrogel microparticles.

The present invention also provides a use of a composition for preparing medicament in repairing articular cartilage defects, wherein the composition comprises hyaluronic acid hydrogel microparticles.

### BRIEF DESCRIPTION OF THE DRAWINGS

Figure 1 is a flowchart of a method for preparing methacrylated hyaluronic acid (Me-HA) hydrogel microparticles.
Figure 2 shows the morphology, size distribution, and mean particle size of the Me-HA hydrogel microparticles.
Figure 3 shows the *in vitro* degradation of methacrylated hyaluronic acid hydrogel microparticles.
Figure 4 shows the O'Driscell histological cartilage repair score of prototypes of methacrylated hyaluronic acid hydrogel microparticles for osteochondral defect repair in a rabbit model.

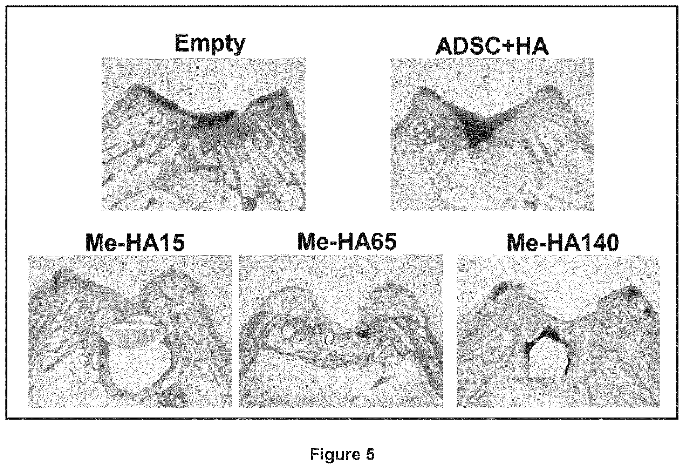

Figure 5 shows the cartilage repair of adipose derived stem cells together with hyaluronic acid and methacrylated hyaluronic acid for osteochondral defect repair in a rabbit model

### DETAILED DESCRIPTION OF THE INVENTION

The present invention provides a method for preparing hyaluronic acid microparticles. After hyaluronic acid is made into hyaluronic acid hydrogel by photo-crosslinking, the hyaluronic acid hydrogel is chopped into hyaluronic acid microparticles to adjust the degradation rate of the hyaluronic acid microparticles which can be used alone and effectively to repair articular cartilage defects without the chondrocytes or mesenchymal stem cells.

The present invention provides a method for preparing hyaluronic acid hydrogel microparticles, comprising: (a) reacting hyaluronic acid with methacrylic anhydride to synthesize a methacrylated hyaluronic acid conjugate; (b) mixing the methacrylated hyaluronic acid conjugate with a photoinitiator, and irradiating ultraviolet light to carry out a photopolymerization reaction to obtain a hyaluronic acid hydrogel; and (c) passing the hyaluronic acid hydrogel through a sieve to obtain the hyaluronic acid hydrogel microparticles.

In one embodiment, the photoinitiator is 2-methyl-1-[4-(hydroxyethoxy)phenyl]-2-methyl- 1-propanone.

In one embodiment, the mesh size of the sieve is from 10 to 500 µm.

In one embodiment, the particle size of hyaluronic acid hydrogel microparticles is from 1 to 300 µm. In another embodiment, the particle size of the hyaluronic acid hydrogel microparticles is from 70 to 200 µm. In another embodiment, the particle size of the hyaluronic acid hydrogel microparticles is from 100 to 150 µm.

In one embodiment, the degree of esterification of the hyaluronic acid hydrogel microparticles is from 5% to 2000%. In another embodiment, the degree of esterification of the hyaluronic acid hydrogel microparticles is from 15% to 140%.

The present invention also provides a use of a composition for preparing medicament in repairing articular cartilage defects, wherein the composition comprises hyaluronic acid hydrogel microparticles.

In one embodiment, the hyaluronic acid hydrogel microparticles are prepared by: (a) reacting hyaluronic acid with methacrylic anhydride to synthesize a methacrylated hyaluronic acid conjugate; (b) mixing the methacrylated hyaluronic acid conjugate with a photoinitiator, and irradiating ultraviolet light to carry out a photopolymerization reaction to obtain a hyaluronic acid hydrogel; and (c) passing the hyaluronic acid hydrogel through a sieve to obtain the hyaluronic acid hydrogel microparticles.

The present invention also provides a method for repairing articular cartilage defects, which comprises administering hyaluronic acid hydrogel microparticles to a part of an articular cartilage defect.

In one embodiment, the hyaluronic acid hydrogel microparticles are prepared by: (a) reacting hyaluronic acid with methacrylic anhydride to synthesize a methacrylated hyaluronic acid conjugate; (b) mixing the methacrylated hyaluronic acid conjugate with a photoinitiator, and irradiating ultraviolet light to carry out a photopolymerization reaction to obtain a hyaluronic acid hydrogel; and (c) passing the hyaluronic acid hydrogel through a sieve to obtain the hyaluronic acid hydrogel microparticles.

The term "Hydrogel" as used herein refers to a gel with water as a dispersion medium, which is a crosslinked polymer formed by introducing a portion of hydrophobic groups and hydrophilic residues in a water-soluble polymer having a cross-linking network structure, so that the hydrophilic residues are combined with water molecules to connect the water molecule to the interior of the network, and the hydrophobic residues swell when being in contact with water. Therefore, all water-soluble or hydrophilic polymers can form a hydrogel through certain chemical crosslinking or physical crosslinking. The hydrogel can be classified as physical gel and chemical gel. (1) The physical gel is formed by physical acting forces such as electrostatic interaction, hydrogen bond, chain entanglement and the likes. The gel is non-permanent and can be converted into a solution by heating the gel, so the gel is also called pseudogel or a thermoreversible gel. Many natural polymers are in a stable gel state at normal temperature, such as k2 carrageenan, agar and the like. In the synthetic polymer, polyvinyl alcohol (PVA) is a typical example, after freezing-thawing treatment, a hydrogel stable below 60°C can be obtained. Further, (2) the chemical gel is a three-dimensional network polymer formed by crosslinking chemical bonds, which is permanent and is also called true gel. Based on the difference in the size and the shape, the hydrogel can be classified into macro-gel and the micro-gel (microparticle). Based on the difference in shape, the macro-gel can be further classified into columnar, porous sponge, fibrous, film, spherical macrogel and the like, and the currently prepared micro-particles are classified as of micro-scale and nano-scale.

### EXAMPLES

The present invention may be implemented in different forms and is not limited to the examples described in the following text. The following embodiments are merely representative of different aspects and characteristics of the present invention.

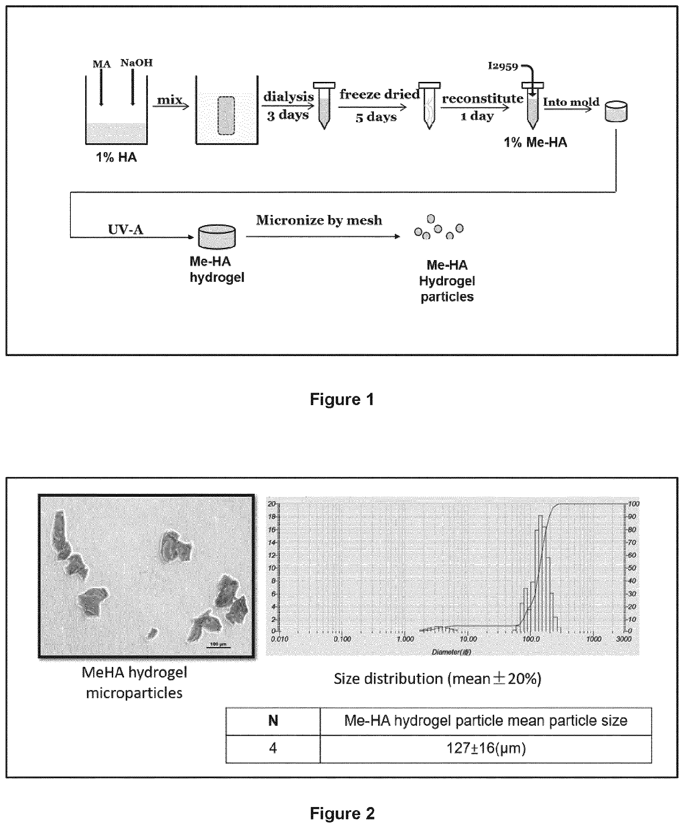

The preparation process of hyaluronic acid hydrogel microparticles is shown in Figure 1. Methacrylated hyaluronic acid (Me-HA) conjugates were synthesized by adding methacrylic anhydride to a 1% (w/v) of HA solution, wherein the pH value of the 1% of HA solution was adjusted to be 8 in deionized distilled water with 5 N sodium hydroxide. For purification, the macromer solution was dialysed against deionized distilled water for at least 72 h and the final product was obtained by lyophilization. The degree of substitution was determined by using proton nuclear magnetic resonance spectroscopy (1 H NMR), which was defined as the number of methacryloyl groups in each disaccharide repeat unit, and calculated from the ratio of the relative peak integration of the methacrylate protons (peaks at ∼5.2 and ∼5.5 ppm) and the methyl protons of HA (∼4.3 ppm). Regarding the formation of the hydrogel, Me-HA was redissolved at a concentration of 1% (w/v) in PBS containing 0.05% (w/v) of 2-methyl-1-[4-(hydroxyethoxy)phenyl]-2-methyl-1-propanone (Irgacure 2959, 12959). The Me-HA solution was photopolymerized with 0.3 J cm2 of ultraviolet light (365 nm, Spectroline, USA). The Me-HA hydrogel was pressed through a 100 µm mesh to be micronized. The micronized Me-HA hydrogel microparticles were filtered by a 200 µm mesh. The Me-HA hydrogel microparticles having a size under 200 µm were collected. Then the filtered Me-HA hydrogel microparticles were filtered through a 70 µm mesh, and those having a size above 70 µm were collected for further experiments.

The morphology, size distribution and mean particle size of the hyaluronic acid hydrogel microparticles are shown in Figure 2. The Me-HA hydrogel microparticles were irregular in shape, and the mean particle size of the Me-HA hydrogel microparticles was 127±16 µm.

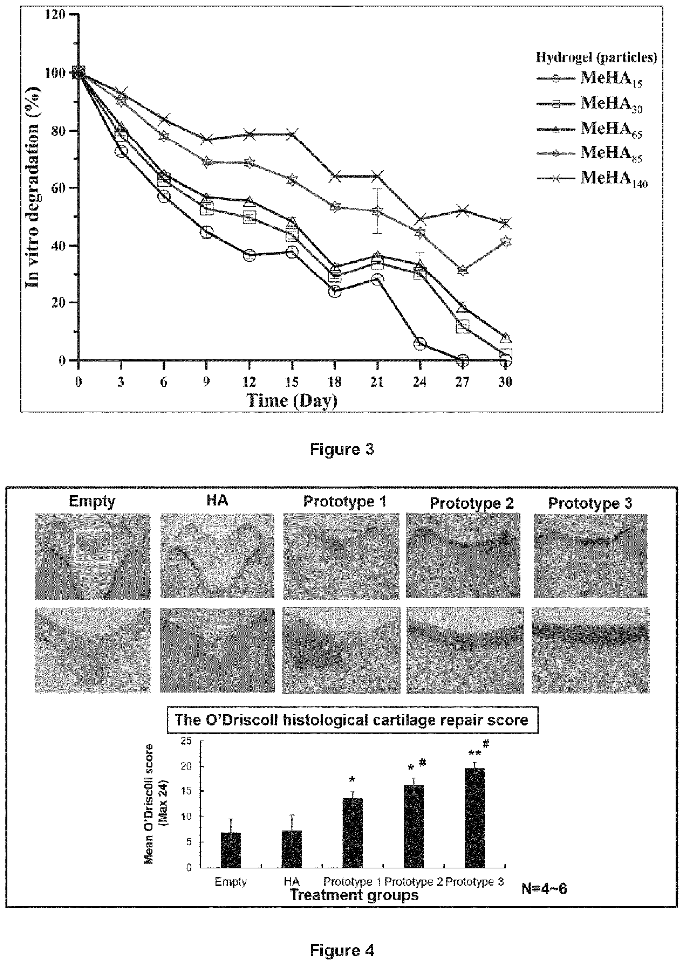

The Me-HA hydrogel microparticles were subjected to an in vitro degradation test, and the degradation results are shown in Figure 3, wherein 15, 30, 65, 85, and 140 at the bottom right of Me-HA represented the degree of methylacrylation. Me-HA15: the degree of methylacrylation of Me-HA hydrogel particles is 15%. Me-HA₃₀: the degree of methylacrylation of the Me-HA hydrogel microparticles was 30%. Me-HA₆₅: the degree of methylacrylation of the Me-HA hydrogel microparticles was 65%. Me-HA₈₅: the degree of methylacrylation of the Me-HA hydrogel microparticles was 85%. Me-HA₁₄₀: the degree of methylacrylation of the Me-HA hydrogel microparticles was 140%.

The osteochondral defect repair in a rabbit model was used in the experiments, the O'Driscell histological cartilage repair score was obtained after the animal model was treated with methacrylated hyaluronic acid hydrogel microparticles.

The groups were as follows: the Empty group: untreated; the HA group: the osteochondral defects were treated with HA; the Prototype 1 group: the osteochondral defects were treated with the Me-HA₁₅ hydrogel microparticle; the Prototype 2 group: the osteochondral defects were treated with the Me-HA₆₅ hydrogel microparticle; the Prototype 3 group: the osteochondral defects were treated with the Me-HA₁₄₀ hydrogel microparticle. *p<0.05; **p<0.01: being compared with the Empty group, n=4-6. # p<0.05: being compared with the HA group, n=4-6. The results showed that the repairing effect of the osteochondral defects in the group treated with the Me-HA was significantly higher than that of the untreated group or the HA treated group.

The animal model was treated with adipose derived stem cells (ADSC) and hyaluronic acid (HA) and Me-HA for cartilage repair experiments. The groups were as follows: the Empty group: untreated; the ADSC+HA group: the osteochondral defects were treated with adipose derived stem cells with HA; the Me-HA₁₅ group: the osteochondral defects were treated with Me-HA₁₅ hydrogel without being micronized; the Me-HA₆₅ group: the osteochondral defects were treated with Me-HA₆₅ hydrogel without being micronized, and the Me-HA₁₄₀ group: the osteochondral defects were treated with Me-HA₁₄₀ hydrogel without being micronized. The results showed that the Me-HA could effectively repair the articular cartilage defects without using chondrocytes or mesenchymal stem cells.

Those skilled in the art recognize the foregoing outline as a description of the method for communicating hosted application information. The skilled artisan will recognize that these are illustrative only and that many equivalents are possible.

## Claims

1. A method for preparing hyaluronic acid hydrogel microparticles, comprising:
(a) reacting hyaluronic acid with methacrylic anhydride to synthesize a methacrylated hyaluronic acid conjugate;
(b) mixing the methacrylated hyaluronic acid conjugate with a photoinitiator, and irradiating ultraviolet light to carry out a photopolymerization reaction to obtain a hyaluronic acid hydrogel; and
(c) passing the hyaluronic acid hydrogel through a sieve to obtain the hyaluronic acid hydrogel microparticles.

2. The method of claim 1, wherein the photoinitiator is 2-methyl-1-[4-(hydroxyethoxy)phenyl]- 2-methyl-1-propanone.

3. The method of claim 1, wherein the mesh size of the sieve is from 10 to 500 µm.

4. The method of claim 1, wherein the particle size of the hyaluronic acid hydrogel microparticles is from 1 to 300 µm.

5. The method of claim 1, wherein the particle size of the hyaluronic acid hydrogel microparticles is from 70 to 200 µm.

6. The method of claim 1, wherein the particle size of the hyaluronic acid hydrogel microparticles is from 100 to 150 µm.

7. The method of claim 1, wherein the degree of esterification of the hyaluronic acid hydrogel microparticles is from 15 % to 140 %.

8. A use of a composition for preparing medicament in repairing articular cartilage defects, wherein the composition comprises hyaluronic acid hydrogel microparticles.

9. The use of claim 8, wherein the hyaluronic acid hydrogel microparticles are prepared by:
(a) reacting hyaluronic acid with methacrylic anhydride to synthesize a methacrylated hyaluronic acid conjugate;
(b) mixing the methacrylated hyaluronic acid conjugate with a photoinitiator, and irradiating ultraviolet light to carry out a photopolymerization reaction to obtain a hyaluronic acid hydrogel; and
(c) passing the hyaluronic acid hydrogel through a sieve to obtain the hyaluronic acid hydrogel microparticles.

10. The use of claim 8, wherein the particle size of the hyaluronic acid hydrogel microparticles is from 100 to 150 µm.
